# EUROPÄISCHE PATENTSCHRIFT

(11) **EP 2 638 002 B1**
(45) Veröffentlichungstag und Bekanntmachung des Hinweises auf die Patenterteilung: **10.05.2017**
(21) Anmeldenummer: 11781782.5
(22) Anmeldetag: 07.11.2011
(51) Int. Cl.: C07C 209/08, C07C 209/62, C07C 211/15, C07C 211/27

(54) **VERFAHREN ZUR HERSTELLUNG VON 2,2-DIFLUORETHYLAMIN AUSGEHEND VON EINER BENZYLAMIN-VERBINDUNG**
METHOD FOR PREPARING 2,2-DIFLUOROETHYLAMINE STARTING FROM A BENZYLAMINE COMPOUND
PROCÉDÉ DE PRODUCTION DE 2,2-DIFLUORO-ÉTHYLAMINE À PARTIR D'UN COMPOSÉ BENZYLAMINE

(30) Priorität: 12.11.2010 US 413045 P; 12.11.2010 EP 10191061
(43) Veröffentlichungstag der Anmeldung: 18.09.2013
(73) Patentinhaber: Bayer Intellectual Property GmbH, 40789 Monheim am Rhein (DE)
(72) Erfinder: LUI, Norbert, 51519 Odenthal (DE); HEINRICH, Jens-Dietmar, 51381 Leverkusen (DE); FUNKE, Christian, 42799 Leichlingen (DE); SCHLEGEL, Günter, 51381 Leverkusen (DE); MÜLLER, Thomas Norbert, 40789 Monheim (DE)
(74) Vertreter: BIP Patents
(86) Internationale Anmeldenummer: PCT/EP2011/069545
(87) Internationale Veröffentlichungsnummer: WO 2012/062702

(56) Entgegenhaltungen:
- WO-A1-2009/036901
- DICKEY ET AL: "Fluorinated Aminoanthraquinone dyes", INDUSTRIAL AND ENGINEERING CHEMISTRY PRODUCT RESEARCH AND DEVELOPMENT, AMERICAN CHEMICAL SOCIETY, EASTON, PA, US, Bd. 48, Nr. 2, 1. Januar 1956 (1956-01-01) , Seiten 209-213, XP002559402, ISSN: 0536-1079, DOI: DOI:10.1021/IE50554A018 in der Anmeldung erwähnt
- AOKI K ET AL: "Enantioselective Deprotonation of 4-tert-Butylcyclohexanone by Fluorine-Containing Chiral Lithium Amides Derived from alpha-Phenethylamine", TETRAHEDRON LETTERS, ELSEVIER, AMSTERDAM, NL, Bd. 38, Nr. 14, 7. April 1997 (1997-04-07) , Seiten 2505-2506, XP004056744, ISSN: 0040-4039, DOI: DOI:10.1016/S0040-4039(97)00378-X

## Beschreibung

Die vorliegende Erfindung betrifft ein Verfahren zur Herstellung von 2,2-Difluorethylamin durch die Umsetzung einer Benzylamin-Verbindung mit 2,2-Difluor-1-halogenethan.

2,2-Difluorethylamin ist eine wichtige Zwischenverbindung bei der Wirkstoffherstellung. Es sind verschiedene Herstellungsmethoden für 2,2-Difluorethylamin bekannt (z.B. WO 2009/036901).

Donetti et al. (J. Med. Chem. 1989, 32, 957-961) beschreiben die Synthese von 2,2-Difluorethylamin-Hydrochlorid ausgehend von 2,2-Difluoracetamid. Hierbei wird mit einer Diboran-Lösung in Tetrahydrofuran (THF) das gewünschte Amin hergestellt. Die Ausbeute beträgt 48%.

Kluger et al. (JACS 1982, 104, 10, Seiten 2891-2897) beschreiben die Synthese von 2,2-Difluorethylamin ausgehend vom Amid mit Natriumboranat und Bortrifluoridetherat. Die Ausbeute beträgt 60%. Vyazkov, V.A. et al. (Vyazkov, V. A.; Gontar, A. F.; Grinevskaya, V. K.; Igoumnova, E. V.; Igoumnov, S. M. A. N. Nesmeyanov Institute of Organoelement Compounds, Russian Academy of Sciences, Moscow, Russia Fluorine Notes (2009), 65) beschreiben ebenfalls die Reduktion mit Natriumboranat in einer Ausbeute von 50 - 65 %.

Auch Kollonitsch (US 4,030,994) beschreibt eine Synthese von 2,2-Difluorethylamin, nämlich die Umsetzung von Ethylamin mit Fluoroxytrifluormethan in Fluorwasserstoff unter UV Bestrahlung.

Swarts beschreibt in seinem Artikel mit dem Titel "Über einige fluorhaltige-Alkylamine" (Chem. Zentralblatt, Band 75, 1904, Seiten 944-945) die Herstellung von 2,2-Difluorethylamin und von Tetrafluorethylamin, mit anschließender Abtrennung der beiden Produkte durch fraktionierte Destillation oder als Chlorhydrat- oder Oxalat-Salze nach vorheriger Umwandlung der erhaltenen Produkte. Swarts verwendet als Ausgangsverbindung 1-Brom-2,2-difluorethan und erhitzt dieses über einen verhältnismäßig langen Zeitraum, nämlich 3 Tage, im Reaktionsrohr mit 2 Mol-Alkoholischem Ammoniak auf relativ hohe Temperaturen, nämlich 125° - 145 °C. Die Ausgangsverbindung wird vollständig in die Verbindungen Difluorethylamin und Tetrafluorethylamin umgesetzt.

Aoki et al. (Tetrahedron Letters, Elsevier, Amsterdam, NL, Bd. 38, Nr. 14, 7. April 1997 (1997-04-07), Seiten 2505 - 2506, XP004056744, ISSN 0040-4039) beschreibt ein Verfahren zur Herstellung einer N-Benzyl-2,2-Difluorethanamin-Verbindung der Formel (IV), bei dem die Benzylaminverbindung acyliert und das entstandene Amid dann reduziert wird.

Die Herstellung von 2,2-Difluorethylamin wird auch bei Dickey et al. (Industrial and Engineering Chemistry 1956, Nr. 2, 209-213) beschrieben. 2,2-Difluor-1-chlorethan wird dort mit 28 %-igem Ammoniumhydroxid, d.h. 28 %-iger wässriger Ammoniak-Lösung, in einem Autoklaven (rocking autoclave) zur Reaktion gebracht. Die Reaktionsmischung wird für 31 Stunden auf Temperaturen von 135° bis 140 °C erhitzt. Nach Beendigung der Reaktion wird die Reaktionsmischung filtriert und das Amin vom Reaktionsgemisch abdestilliert. Da sich aber noch eine Menge Ammoniak und etwas Wasser im Destillat befindet, wird das Amin über Natriumhydroxid getrocknet und nochmals destilliert. Das

Amin wurde so in einer Ausbeute von 65 % erhalten.

Dieses Verfahren ist nachteilig, denn es benötigt - genauso wie das Verfahren nach Swarts - eine sehr lange Reaktionszeit von 31 Stunden und die Ausbeute von 65 % ist eher gering. Gleichzeitig ist die Reaktionsmischung stark korrodierend, da der wässrige Ammoniak in Kombination mit den im Reaktionsgemisch vorhandenen Chlorid- und Fluoridionen bei den im Verfahren verwendeten hohen Temperaturen metallische Werkstoffe angreift.

Alle diese bekannten Verfahren sind nachteilig, insbesondere weil sie sich nicht im wirtschaftlich sinnvollen großtechnischen (industriellen) Maßstab durchführen lassen. Die geringe Ausbeute und der Einsatz von teuren und gefährlichen Chemikalien wie z.B. Natriumboranat/BF₃ oder Diboran verhindern, dass die Verfahren nach Donetti et al. und Kluger et al. für die großtechnische Herstellung von 2,2-Difluorethylamin geeignet sind. Das Verfahren nach Kollonitsch et al. verwendet gefährliche Chemikalien und es wird kein reines 2,2-Difluorethylamin erhalten. Das Verfahren nach Dickey et al. und das Verfahren nach Swarts sind ebenfalls für den großtechnischen Einsatz ungeeignet bzw. unwirtschaftlich, denn sie benötigen sehr lange Reaktionszeiten und sind gleichzeitig nicht selektiv, so dass die Ausbeuten der Verfahren unbefriedigend sind.

Weiterhin ist der Einsatz von Ammoniak bei hohen Temperaturen problematisch, da man spezielle druckfeste Apparaturen benötigt, was sicherheitstechnisch anspruchsvoll und teuer ist.

Ausgehend von den bekannten Verfahren zur Herstellung von 2,2-Difluorethylamin stellt sich nun die Aufgabe, wie 2,2-Difluorethylamin einfach und kostengünstig hergestellt werden kann. Unter kostengünstigen Verfahren werden solche Verfahren verstanden, die ohne großen finanziellen Aufwand durchzuführen sind, weil die Ausgangsstoffe beispielsweise ungefährlich sind, keine sonstigen technischen Probleme auftauchen, beispielsweise weil das Reaktionsgemisch korrodierend wirkt, und/oder das gewünschte 2,2-Difluorethylamin in einer ausreichend hohen Ausbeute und Reinheit erhalten wird, weil etwa die Reaktion weitgehend selektiv verläuft.

Es wurde nun ein besonders vorteilhaftes Verfahren zur Herstellung von 2,2-Difluorethylamin gefunden, mit dem die oben genannten Nachteile vermieden werden und das einfach im großtechnischen Maßstab einzusetzen ist. Im erfindungsgemäßen Verfahren wird in einem ersten Schritt eine 2,2-Difluor-1-halogenethan-Verbindung unter vergleichsweise milden Reaktionsbedingungen und vergleichsweise kurzer Reaktionszeit selektiv zur gewünschten N-Benzyl-2,2-difluorethanamin-Verbindung umgesetzt. In einem zweiten Schritt wird die Benzylgruppe durch katalytische Hydrierung wieder entfernt und das gewünschte N,N-Difluorethylamin entsprechend erhalten.

Gegenstand der Erfindung ist somit ein Verfahren zur Herstellung von 2,2-Difluorethylamin der Formel (I)

CHF₂CH₂NH₂ (I)

das die folgenden Schritte (i) und (ii) umfasst:
Schritt (i) --Alkylierung: Umsetzung von 2,2-Difluor-1-halogenethan der allgemeinen Formel (II)

   CHF₂-CH₂Hal (II),

   worin Hal für Chlor, Brom oder Jod steht, Hal steht bevorzugt für Chlor oder Brom, ganz bevorzugt für Chlor,
   mit einer Benzylamin-Verbindung der Formel (III) 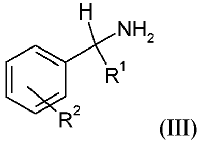 worin
   R¹ für Wasserstoff oder für gegebenenfalls mit Halogen substituiertes C₁-C₁₂-Alkyl steht, bevorzugt steht R¹ für Wasserstoff oder C₁-C₆-Alkyl, und
   R² für Wasserstoff, Halogen, gegebenenfalls mit Halogen substituiertes C₁-C₁₂-Alkyl, oder gegebenenfalls mit Halogen substituiertes C₁-C₆-Alkoxy steht, bevorzugt steht R² für Wasserstoff, Fluor, Chlor, C₁-C₆-Alkyl oder C₁-C₃-Alkoxy (insbesondere Methoxy), zu einer N-Benzyl-2,2-difluorethanamin-Verbindung der Formel (IV)

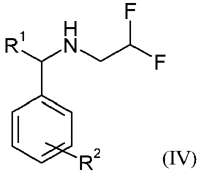

   worin R¹ und R² wie oben definiert sind,
   in Gegenwart einer organischen oder anorganischen Base, die in der Lage ist, die frei werdenden Halogenwasserstoff-Verbindungen zu binden;
   und
Schritt (ii): Katalytische Hydrierung der im Schritt (i) erhaltenen N-Benzyl-2,2-difluorethanamin-Verbindung der Formel (IV) zu 2,2-Difluorethylamin der Formel (I) oder einem Salz davon. Das erfindungsgemäße Verfahren kann durch das folgende Schema veranschaulicht werden:

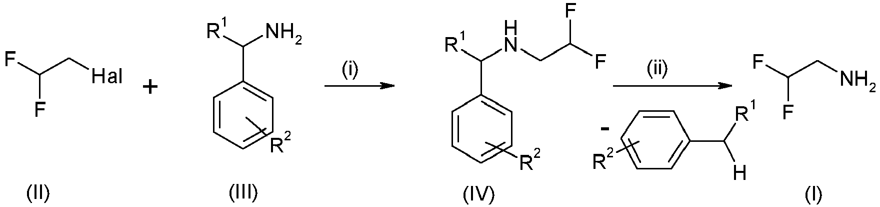

Das gewünschte 2,2-Difluorethylamin wird mit dem erfindungsgemäßen Verfahren mit guten Ausbeuten, kurzen Reaktionszeiten und in hoher Reinheit erhalten, weshalb eine umfangreiche Aufarbeitung des unmittelbaren Reaktionsprodukts im Allgemeinen nicht erforderlich ist.

Gegenstand der Erfindung ist gleichfalls das Verfahren des Schritts (i) zur Herstellung von N-Benzyl-2,2-difluorethanamin der Formel (IV) 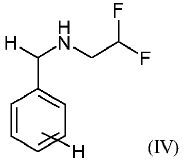 umfassend die Umsetzung von 2,2-Difluor-1-chlorethan mit Benzylamin der Formel (III) 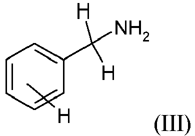 in Gegenwart einer organischen oder anorganischen Base, das die für Schritt (i) beschriebenen Verfahrensschritte, Reaktionsbedingungen und Reaktionspartner beinhaltet. Ebenfalls beschrieben ist die Verwendung von N-Benzyl-2,2-difluorethanamin der Formel (IV) zur Herstellung von 2,2-Difluorethylamin, das die für Schritt (ii) beschriebenen Verfahrensschritte, Reaktionsbedingungen und Reaktionspartner beinhaltet.

Obwohl aus M. Hudlicky in "Chemistry of Organofluorine Compounds", 2. Auflage, 1976, S. 489-490 und Houben Weyl, E 10b/2, S. 92-98 bekannt ist, dass 2,2-Difluor-1-halogenethan unter basischen Bedingungen unter Abspaltung von HHal (HCl, HBr oder HJ) zu Vinylidenfluorid reagiert und es dadurch für die Umsetzung in Schritt (i) nicht mehr zur Verfügung steht, und obwohl aus J. Org. Chem. 2007, 72 (22) S. 8569 bekannt ist, dass 2,2-Difluorethylamine sehr reaktiv sind und es sehr wahrscheinlich ist, dass die erhaltene N-Benzyl-2,2-difluorethanamin-Verbindung der Formel (IV) unter den Reaktionsbedingungen im Schritt (i) weiterreagiert, haben die Erfinder überraschenderweise gefunden, dass durch Schritt (i) des erfindungsgemäßen Verfahrens die N-Benzyl-2,2-difluorethanamin-Verbindung der Formel (IV) in guter Ausbeute und Reinheit erhalten wird, so dass eine umfangreiche Aufreinigung entfallen kann. Letztendlich wird deshalb auch die Zielverbindung 2,2-Difluorethylamin in einer sehr guter Ausbeute bezogen auf die im Schritt (i) eingesetzten Edukte erhalten.

Im Hinblick auf die Alkylierung im Schritt (i) haben die Erfinder - entgegen der Erwartung, dass vermehrt Doppel- oder Mehrfachalkylierungen auftreten - gefunden, dass wenn die Summe der molaren Mengen von umzusetzender Benzylamin-Verbindung der Formel (III) und Säurefänger kleiner ist als die molare Menge an eingesetzten 2,2-Difluorhalogenethan der Formel (II), sehr hohe Ausbeuten erzielt werden. Wird die Benzylamin-Verbindung der Formel (III) sowohl als Edukt als auch als Säurefänger eingesetzt, so gilt auch hier, dass die Summe der molaren Menge an Benzylamin-Verbindung der Formel (III) die umgesetzt wird und der molaren Menge an Benzylamin-Verbindung der Formel (III), die als Säurefänger dient, geringer ist als die molare Menge an eingesetztem 2,2-Difluorhalogenethan der Formel (II).

Im erfindungsgemäßen Verfahren werden bevorzugt 2,2-Difluor-1-halogenethan-Verbindungen der Formel (II) eingesetzt, in denen Hal für Chlor oder Brom steht. Besonders bevorzugt wird die Verbindung 2,2-Difluor-1-chlorethan (CHF₂-CH₂Cl) eingesetzt.

Im erfindungsgemäßen Verfahren werden weiterhin bevorzugt Benzylamin-Verbindungen der Formel (III) eingesetzt, in denen
(a) R¹ ausgewählt ist aus einer Gruppe bestehend aus Wasserstoff, Methyl, Ethyl und n- oder i-Propyl und in der R² ausgewählt ist aus einer Gruppe bestehend aus Wasserstoff, Methyl, Chlor und Methoxy; oder
(b) R¹ ausgewählt ist aus einer Gruppe bestehend aus Wasserstoff und Methyl und R² ausgewählt ist aus einer Gruppe bestehend aus Wasserstoff, Methyl und Chlor; oder
(c) R¹ und R² jeweils für Wasserstoff stehen.

Benzylamin-Verbindungen der Formel (III) sind bekannt. Sie können nach bekannten Verfahren hergestellt werden und sind zum Teil auch käuflich erhältlich.

Es ist besonders bevorzugt, 2,2-Difluor-1-chlorethan als Verbindung der Formel (II) mit einer Benzylamin-Verbindung der Formel (III), in der die Reste R¹ und R² die unter einem der Punkte (a), (b) oder (c) genannten Bedeutungen haben im erfindungsgemäßen Verfahren umzusetzen.

Sofern nichts anderes angegeben, bezieht sich die Bezeichnung "Alkyl" - in Alleinstellung oder in Kombination mit anderen Begriffen, wie beispielsweise Alkoxy - auf lineare oder verzweigte gesättigte Kohlenwasserstoffketten mit bis zu 12 Kohlenstoffatomen, d.h. C₁-C₁₂-Alkyl, bevorzugt mit bis zu 6 Kohlenstoffen, d.h. C₁-C₆-Alkyl, besonders bevorzugt mit bis zu 4 Kohlenstoffen, d.h. C₁-C₄-Alkyl. Beispiele solcher Alkyle sind Methyl, Ethyl, n-Propyl oder Isopropyl, n-, i-, s- oder t-Butyl, n-Pentyl, n-Hexyl, n-Heptyl, n-Octyl, n-Nonyl, n-Decyl, n-Undecyl und n-Dodecyl. Die Alkyle können mit Halogen substituiert sein. Sofern nichts anderes angegeben steht "Halogen" oder "Hal" für Fluor, Chlor, Brom oder Jod.

Die Umsetzung von 2,2-Difluor-1-halogenethan der Formel (II) mit der Benzylamin-Verbindung der Formel (III) aus Schritt (i) kann in Substanz, d.h. ohne Hinzufügen eines Lösungsmittels oder in Gegenwart eines Lösungsmittels durchgeführt werden.

Im Falle dass in Schritt (i) ein Lösungsmittel zum Reaktionsgemisch hinzugefügt wird, wird es vorzugsweise in einer solchen Menge eingesetzt, dass das Reaktionsgemisch während des ganzen Verfahrens gut rührbar bleibt. Vorteilhafterweise wird, bezogen auf das Volumen des eingesetzten 2,2-Difluor-1-halogenethan, die 1- bis 50-fache Lösungsmittelmenge, bevorzugt die 2- bis 40-fache Lösungsmittelmenge, besonders bevorzugt die 2- bis 20-fache Lösungsmittelmenge verwendet. Unter Lösungsmittel werden erfindungsgemäß auch Gemische reiner Lösungsmittel verstanden. Geeignete Lösungsmittel sind alle unter den Reaktionsbedingungen inerten organischen Lösungsmittel. Erfindungsgemäß geeignete Lösungsmittel sind insbesondere Wasser, Ether (z.B. Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Diethylenlykoldimethylether, Triethylenglykoldimethylether, Tetrahydrofuran, 2-Methyltetrahydrofuran, Dioxan, und Polyether des Ethylenoxids und/oder Propylenoxids); Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Benzol, Toluol, Xylol); halogenierte Aromate (z.B. Chlorbenzol, Dichlorbenzol); Amide (z.B. Hexamethylphosphorsäuretriamid, Formamid, N,N-Dimethylacetamid, *N-*Methyl-formamid, *N*,*N-*Dimethyl-formamid, *N*,*N-*Dipropyl-formamid, *N*,*N-*Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N*,*N'-*1,4-Diformyl-piperazin); Nitrile (z.B. Acetonitril, Propionitril, n- oder iso-Butyronitril oder Benzonitril); Ketone (z.B. Aceton) oder Gemische davon.

Bevorzugte Lösungsmittel in Schritt (i) sind aromatische und/oder aliphatische Kohlenwasserstoffe, insbesondere Toluol, N,N-Dimethylacetamid, Tetramethylensulfoxid und *N-*Methyl-pyrrolidon.

Es ist erfindungsgemäß bevorzugt Schritt (i) in Substanz, d.h. ohne Lösungsmittel durchzuführen. Hierdurch kann das Verfahren noch kostengünstiger durchgeführt werden, weil die Lösungsmittel weder gekauft, noch nach Reaktion entsorgt werden müssen.

Die Umsetzung des Schritts (i) erfolgt in Gegenwart eines oder mehrerer Säurefänger, die in der Lage sind, die bei der Reaktion frei werdende Halogenwasserstoff-Verbindung (d.h. HCl, HBr, HI) zu binden.

Geeignete Säurefänger sind alle organischen und anorganischen Basen, die in der Lage sind, die frei werdenden Halogenwasserstoff-Verbindungen zu binden. Beispiele für organische Basen sind tertiäre Stickstoffbasen, wie z.B. tertiäre Amine, substituierte oder unsubstituierte Pyridine und substituierte oder unsubstituierte Chinoline, Triethylamin, Trimethylamin, Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethylendiamin, N,N-Dimethyl-1,4-diazacyclohexan, N,N-Di-ethyl-1,4-diazacyclohexan, 1,8-Bis-(Dimethylamino)naphthalin, Diazabicyclooctan (DABCO), Diazabicyclononan (DBN), Diazabicycloundecan (DBU), Butylimidazol und Methylimidazol.

Beispiele für anorganische Basen sind Alkali- oder Erdalkalimetallhydroxyde, Hydrogencarbonate oder Carbonate und sonstige anorganische wässrige Basen, bevorzugt sind z.B. Natriumhydroxid, Kaliumhydroxid, Lithiumhydroxid, Calciumhydroxid, Natriumcarbonat, Kaliumcarbonat, Natriumhydrogencarbonat, Kaliumhydrogencarbonat und Natriumacetat.

Das molare Verhältnis von Säurefänger, insbesondere von oben genannten Basen, zur eingesetzten Benzylamin-verbindung der Formel (III) liegt im Bereich von etwa 0,1 bis etwa 3, bevorzugt im Bereich von etwa 0,5 bis etwa 3, besonders bevorzugt im Bereich von etwa 0,7 bis etwa 1,3. Der Einsatz größerer Mengen an Base ist technisch möglich, führt jedoch zu einem Ausbeuteverlust.

Das molare Verhältnis von 2,2-Difluor-1-halogenethan zum eingesetzten Amin der Formel (III) liegt normalerweise im Bereich von etwa 30 : 1 bis etwa 1 : 3, bevorzugt im Bereich von etwa 10 : 1 bis etwa 1 : 2, besonders bevorzugt im Bereich von etwa 8 : 1 bis etwa 1:1.

In einer bevorzugten Ausführungsform dient die Benzylamin-Verbindung der Formel (III) als Säurefänger, so dass kein weiterer Säurefänger zugesetzt werden muss. In diesem Fall liegt das molare Verhältnis von 2,2-Difluor-1-halogenethan zum eingesetzten Amin der Formel (III) normalerweise im Bereich von etwa 15 : 1 bis etwa 1 : 3, bevorzugt im Bereich von etwa 8 : 1 bis etwa 1 : 2,5, besonders bevorzugt im Bereich von etwa 4 : 1 bis etwa 1:2.

Das Benzylamin der Formel (III) und die Base können zu dem 2,2-Difluor-1-halogenethan der Formel (II) auch zudosiert werden.

Obwohl Schritt (i) des erfindungsgemäßen Verfahrens im Allgemeinen ohne Zusatz eines Katalysators durchgeführt wird, können im Schritt (i) auch Katalysatoren, die die Umsetzung der Benzylamin-Verbindung der Formel (III) mit 2,2-Difluorhalogenethan beschleunigen, verwendet werden. Durch Verwenden eines Katalysators wird die Reaktionstemperatur absenkt, wodurch auch der Eigendruck der Reaktionsmischung abgesenkt wird. Ist der Eigendruck nicht so hoch, kann unter technische einfacheren Bedingungen gearbeitet werden.

Erfindungsgemäß geeignet sind insbesondere Alkalibromide und -jodide (z.B. Natriumjodid, Kaliumjodid, Kaliumbromid); Ammoniumbromid und Ammoniumjodid; Tetraalkylammoniumbromide und -jodide, (z.B. Tetraethylammoniumjodid); bestimmte Phosphoniumhalogenide, wie Tetraalkyl- oder Tetraarylphosphoniumhalogenide (z.B. Hexadecyl-tri-butyl-phosphoniumbromid, Stearyltributylhosphoniumbromid, Tetrabutylphosphoniumbromid, Tetraoctylphosphoniumbromid, Tetraphenylhosphoniumchlorid und Tetraphenylphosphoniumbromid), Tetrakis(dimethylamino)phoshoniumbromid, Tetrakis(diethylamino)phosphoniumbromid, Tetrakis(dipropylamino)phosphoniumchlorid und -bromid; sowie Bis(dimethylamino)[(1,3-dimethylimidazolidin-2-yliden)amino]methyliumbromid. Mischungen geeigneter Katalysatoren sind auch denkbar.

Von den vorgenannten Katalysatoren sind Natriumjodid, Kaliumjodid, Kaliumbromid, Tetrabutylammoniumbromid und Tetraphenylphosphoniumbromid besonders geeignet, die Umsetzung des Schritts (i) zu beschleunigen. Natrium- und Kaliumjodid sind besonders hervorzuheben.

Der Katalysator kann auch *in-situ* erzeugt werden. Beispielsweise durch eine Reaktion von HBr oder HJ mit Ammoniak oder durch Zugabe von sehr reaktiven Alkylbromiden oder -jodiden (z.B. Methyl- oder Ethylbromid oder -jodid) erzeugt werden.

Falls im Schritt (i) ein Katalysator anwesend ist, so wird er, bezogen auf das eingesetzte 2,2-Difluor-1-halogenethan der Formel (II), in einer Konzentration von etwa 0,01 bis etwa 25 Gew.-% verwendet. Höhere Konzentrationen sind grundsätzlich möglich. Bevorzugt wird der Katalysator in einer Konzentration von etwa 0,2 bis etwa 25 Gew.-%, besonders bevorzugt von etwa 0,4 bis etwa 20 Gew.-%, ganz besonders bevorzugt von etwa 0,5 bis etwa 15 Gew.-%. verwendet. Der Katalysator kann aber auch bevorzugt in einer Konzentration von etwa 0,05 bis etwa 3 Gew.-%, von etwa 0,1 bis etwa 10 Gew.-% oder von etwa 0,5 bis etwa 10 Gew.-%. eingesetzt werden.

Die Umsetzung des Schritt (i) wird grundsätzlich unter Eigendruck in einem druckstabilen geschlossenen Versuchsgefäß (Autoklav) durchgeführt. Der Druck während der Reaktion (d.h. der Eigendruck) ist abhängig von der verwendeten Reaktionstemperatur, dem verwendeten 2,2-Difluor-1-halogenethan, dem eingesetzten Katalysator und der Menge an verwendeter Benzylamin-Verbindung der Formel (III). Gleichfalls hängt der Druck auch von dem verwendeten Lösungsmittel ab, falls Lösungsmittel im Schritt (i) anwesend ist. Ist eine Druckerhöhung gewünscht, so kann eine zusätzliche Druckerhöhung durch Zugabe eines Inertgases, wie Stickstoff oder Argon, erreicht werden.

Die Reaktionstemperatur in Schritt (i) kann in Abhängigkeit der verwendeten Ausgangsstoffe variieren. Wird im Schritt (i) kein Katalysator zugefügt, so wird Schritt (i) bei Innentemperaturen (d.h. der Temperatur, die im Reaktionsgefäß vorhanden ist) im Bereich von etwa 70°C bis etwa 200°C durchgeführt. Es ist bevorzugt, dass bei Durchführung des Reaktionsschrittes (i) die Innentemperatur im Bereich von etwa 90 °C bis etwa 150°C, besonders bevorzugt im Bereich von etwa 90°C bis etwa 140°C liegt. Es wurde festgestellt, dass wenn im bevorzugten Temperaturbereich gearbeitet wird, wenig Nebenreaktionen, insbesondere Mehrfachalkylierungen, auftreten.

Wird in Schritt (i) ein Katalysator verwendet, so erniedrigt sich die Reaktionstemperatur der Reaktionsmischung entsprechend. Dem Fachmann ist bekannt, inwieweit sich die Reaktionstemperatur bei Zufügen eines bestimmten Katalysators absenkt und er kann anhand von Routineversuchen oder anhand seines Fachwissens und anhand der vorgenannten Innentemperaturbereiche den für das spezielle Reaktionsgemisch optimalen Reaktions-Innentemperaturbereich finden.

Die Reaktionsdauer der Umsetzung in Schritt (i) ist kurz und liegt im Bereich von etwa 0,5 bis etwa 20 Stunden. Eine längere Reaktionsdauer ist möglich, jedoch wirtschaftlich nicht sinnvoll.

Die Aufarbeitung des Reaktionsgemisches aus Schritt (i) erfolgt entweder durch Filtration und anschließender fraktionierter Destillation oder durch Verwässerung (Zugabe von Wasser in dem ggf. Salze gelöst sind) der Reaktionsmischung und anschließender Phasentrennung und nachfolgender fraktionierter Destillation. Die Base oder das Benzylamin-Verbindung der Formel (III) kann durch Freisetzung mit einer weiteren Base, z.B. Natronlauge, wieder freigesetzt werden und kann entsprechend wieder in den Prozess zurückgeführt werden.

Die N-Benzyl-2,2-difluorethanamin-Verbindung der Formel (IV) wird dann der katalytischen Hydrierung des Schrittes (ii) unterzogen.

Bei der katalytischen Hydrierung der N-Benzyl-2,2-difluorethanamin-Verbindung der Formel (IV) zum 2,2-Difluorethylamin der Formel (I) in Schritt (ii) wird gasförmiger Wasserstoff in das Reaktionsgefäß eingeleitet oder im Reaktionsgefäß *in-situ* durch die Verwendung von Ameisensäure oder Hydrazin sowie deren Derivate oder Salze davon, erzeugt.

Für die katalytische Hydrierung nach Schritt (ii) kann als Katalysator jeder dem Fachmann bekannte, zur katalytischen Hydrierung geeignete Katalysator verwendet werden. In Frage kommen beispielsweise Palladiumkatalysatoren, Platinkatalysatoren, Raney-Nickel-Katalysatoren oder Raney-Cobalt-Katalysatoren, Lindlar-Katalysatoren, Ruthenium- und Rhodiumkatalysatoren. Neben diesen heterogenen Katalysatoren können auch homogene Katalysatoren verwendet werden. Geeignete Katalysatoren enthalten bevorzugt ein oder mehrere Metalle der Gruppen 8 - 10 des Periodensystems, insbesondere ein oder mehrere Metalle ausgewählt unter Eisen, Ruthenium, Osmium, Kobalt, Rhodium, Iridium, Nickel, Palladium und Platin. Die Metalle können in jedweder chemischen Form vorliegen, z.B. elementar, kolloidal, als Salze oder Oxide, zusammen mit Komplexbildner als Chelate, oder als Legierungen, wobei die Legierungen neben den oben angeführten Metalle auch andere Metalle, wie beispielsweise Aluminium beinhalten können. Die Metalle können in geträgerter Form, d.h. auf einen beliebigen, vorzugsweise anorganischen, Träger aufgebracht, vorliegen. Als Träger eigenen sich beispielsweise Kohlenstoff (Graphit, Kohle oder Aktivkohle), Aluminiumoxid, Siliciumdioxid, Zirkondioxid, Calciumcarbonat, Bariumsulfat und Titandioxid. Erfindungsgemäß bevorzugte Katalysatoren enthalten ein oder mehrere Metalle der Gruppen 8 - 10 des Periodensystems auf einem anorganischen Träger. Erfindungsgemäß besonders bevorzugt sind Katalysatoren, die Platin und/oder Palladium beinhalten und gegebenenfalls auf einem anorganischen Träger aufgebracht sind. Solche Katalysatoren sind beispielsweise PtO₂, Pd(OH)₂ auf Aktivkohle (Pearlman Katalysator), Raney-Nickel-Katalysatoren, und Lindlar-Katalysatoren. Die Katalysatoren können sowohl in ihrer wasserfeuchten als auch trockenen Form eingesetzt werden. Der eingesetzte Katalysator wird bevorzugt für mehrere Umsetzungen wiederverwendet.

Im erfindungsgemäßen Verfahren wird der Katalysator, bezogen auf die eingesetzte N-Benzyl-2,2-difluorethanamin-Verbindung der Formel (IV) in einer Konzentration von etwa 0,01 bis etwa 30 Gew.-% verwendet. Bevorzugt wird der Katalysator in einer Konzentration von etwa 0,1 bis etwa 12 Gew.-% verwendet, besonders bevorzugt von etwa 0,1 bis etwa 2,5 Gew.-%.

Die katalytische Hydrierung aus Schritt (ii) wird in Substanz oder in einem geeigneten Lösungsmittel durchgeführt. Wird Schritt (ii) in einem Lösungsmittel durchgeführt, so wird auch hier das Lösungsmittel vorzugsweise in einer solchen Menge eingesetzt, dass das Reaktionsgemisch während des gesamten Verfahrens rührbar bleibt. Vorteilhafterweise wird, bezogen auf die eingesetzte N-Benzyl-2,2-difluorethanamin-Verbindung der Formel (IV) die etwa 1- bis 50-fache Lösungsmittelmenge (v/v), bevorzugt die etwa 2- bis 40-fache Lösungsmittelmenge, besonders bevorzugt die 2- bis 10-fache Lösungsmittelmenge verwendet.

Als Lösungsmittel kommen alle unter den Reaktionsbedingungen inerten organischen Lösungsmittel in Frage. Unter Lösungsmittel werden erfindungsgemäß auch Gemische reiner Lösungsmittel verstanden.

Im Schritt (ii) sind erfindungsgemäß geeignete Lösungsmittel vor allem Wasser, Ether (z.B. Ethylpropylether, Methyl-tert-butylether, *n*-Butylether, Anisol, Phenetol, Cyclohexylmethylether, Dimethylether, Diethylether, Dimethylglycol Diphenylether, Dipropylether, Diisopropylether, Di-*n-*butylether, Diisobutylether, Diisoamylether, Ethylenglycoldimethylether, Isopropylethylether, Diethylenglykoldimethylether, Triethylenglykoldimethylether, Tetrahydrofuran, 2-Methyltetrahydrofuran, Dioxan, und Polyether des Ethylenoxids und/oder Propylenoxids); aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Benzol, Toluol, Xylol); lineare und verzweigte Carbonsäuren (z.B. Ameisensäure, Essigsäure, Propionsäure, Buttersäure und Isobuttersäure) sowie ihre Ester (z.B. Ethylacetat und Butylacetat), Alkohole (z.B. Methanol, Ethanol, Isopropanol, *n*-Butanol und Iso-Butanol) oder Gemische davon.

Bevorzugt sind aromatische oder aliphatische Kohlenwasserstoffe, insbesondere Toluol und Xylol, Methanol, Essigsäure und *n*-Butylacetat.

Im Schritt (ii) kann zur Beschleunigung der katalytischen Hydrierung eine organische oder anorganische Säure zugesetzt werden. Beispiele für organische Säuren sind aliphatische unverzweigte und verzweigte Mono-, Di- und Tricarbonsäuren (z.B. Essigsäure, Isobuttersäure, Oxalsäure, Citronensäure und Trifluoressigsäure); Sulfonsäurederivate (z.B. p-Toluolsulfonsäure, p-Chlorbenzolsulfonsäure, Methansulfonsäure und Trifluormethansulfonsäure). Beispiele für anorganische Säuren sind Salzsäure, Bromwasserstoffsäure, Schwefelsäure und Phosphorsäure.

Das molare Verhältnis von Säure zur eingesetzten N-Benzyl-2,2-difluorethanamin-Verbindung der Formel (IV) liegt im Bereich von etwa 0,001 bis etwa 10, bevorzugt im Bereich von etwa 0,01 bis etwa 5, besonders bevorzugt im Bereicht von etwa 0,02 bis etwa 1. Der Einsatz größerer Mengen an Säure ist grundsätzlich möglich. Die Säure kann bei geeigneter Handhabbarkeit auch als Lösungsmittel verwendet werden.

Die katalytische Hydrierung in Schritt (ii) kann bei Temperaturen im Bereich von etwa 0°C bis etwa 200°C durchgeführt werden. Vorzugsweise liegt die Innentemperatur im Bereich von etwa 20°C bis etwa 150°C, besonders bevorzugt liegt sie im Bereich von etwa 40°C bis 130°C.

Die Reaktionsdauer der katalytischen Hydrierung ist kurz und liegt im Bereich von etwa 0,1 bis 12 Stunden. Eine längere Reaktionsdauer ist möglich, jedoch nicht wirtschaftlich sinnvoll.

Die katalytische Hydrierung kann unter Überdruck (d.h. bis etwa 200 bar) in einem Autoklaven oder bei Normaldruck in einer Wasserstoffgasatmosphäre durchgeführt werden. Insbesondere bei hohen Reaktionstemperaturen kann es hilfreich sein, bei erhöhtem Druck zu arbeiten. Die (zusätzliche) Druckerhöhung kann durch Zuleiten eines Inertgases, wie Stickstoff oder Argon, bewirkt werden. Die erfindungsgemäße Hydrierung erfolgt bevorzugt bei einem Druck im Bereich von etwa 1 bis etwa 100 bar, besonders bevorzugt bei einem Druck im Bereich von etwa 1,5 bis etwa 10 bar.

Die katalytische Hydrierung kann als Pumphydrierung oder im Batchverfahren erfolgen. Bei der Pumphydrierung wird das N-Benzyl-2,2-difluorethanamin-Verbindung der Formel (IV), das in den zuvor beschriebenen Lösungsmitteln gelöst ist oder in Substanz vorliegt, kontinuierlich und im Batchverfahren portionsweise in einen Autoklaven zugefördert (zugegeben). Der Autoklav befindet sich unter Wasserstoffatmosphäre und ist mit mindestens einem der oben genannten Katalysatoren beschickt.

Nach Reaktionsende kann das erhaltene 2,2-Difluorethylamin der Formel (I) durch Destillation aufgereinigt werden. Alternativ kann das 2,2-Difluorethylamin der Formel (I) auch als Salz, z.B. Hydrochlorid, isoliert und gereinigt werden. Das Salz wird durch Zugabe von Säure vor, während oder nach der katalytische Hydrierung erzeugt. Das Salz kann anschließend durch Zugabe von Base wieder freigesetzt werden.

Das 2,2-Difluorethylamin der Formel (I) liegt nach der erfindungsgemäßen Reaktion gewöhnlich in einer solchen Reinheit vor, dass es nach Filtration des Katalysators im Lösungsmittel weiter verwendet werden kann.

Die vorliegende Erfindung wird anhand der nachfolgenden Beispiele näher erläutert, ohne die Erfindung dabei auf diese einzuschränken.

### Herstellungsbeispiele:

### Beispiel 1 - Herstellung von N-Benzyl-2,2-difluorethanamin (Schritt (i))

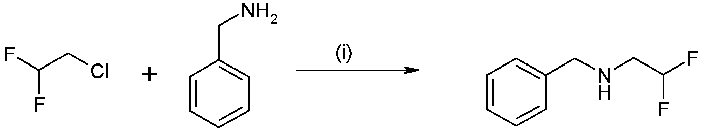

### Beispiel 1.1

1152 g (11,1 mol) 2,2-Difluor-1-chlorethan und 403 g Benzylamin (3,695 mol) werden 16 Stunden bei einer Innentemperatur von 120 °C in einem Autoklaven erhitzt. Anschließend werden 700 g Wasser zugesetzt und die wässrige Phase wird abgetrennt. Die wässrige Phase enthält Benzylaminhydrochlorid, das durch Zugabe von Natronlauge wieder in freies Benzylamin überführt wird. Die organische Phase wird zuerst bei Normaldruck destilliert, wobei das unverbrauchte 2,2-Difluor-1-chlorethan abdestilliert wird. Danach wird noch einmal Vakuumdestilliert (bei etwa 200 mbar), wodurch die restlichen Spuren von 2,2-Difluor-1-chlorethan abdestilliert werden. Man erhält als Destillationsrückstand 306 g N-Benzyl-2,2-difluorethanamin mit einer Reinheit von 98,9 %. Dies entspricht einer Ausbeute von 95,6 % bezogen auf das umgesetzte Benzylamin. Durch erneute Destillation kann N-Benzyl-2,2-difluorethanamin in einer Reinheit von größer 99 % erhalten werden.
NMR ¹H (CDCl₃): 7,24 - 7,35 (m, 5H), 5,84 (tt, 1 H), 3,84 (s, 2H), 2,95 (dt, 2H)

### Beispiel 1.2

2 g (20 mmol) 2,2-Difluor-1-chlorethan und 4,5 g (41 mmol) Benzylamin werden in 62 g N-Methylpyrrolidon 16 Stunden bei einer Innentemperatur von 120°C in einem Autoklaven erhitzt. Anschließend werden 50 g 1N Salzsäure zugesetzt und das Lösungsmittel im Vakuum entfernt. Der Rückstand wird in 50 ml Wasser und 50 ml Dichlormethan aufgenommen und mit festem Natriumhydrogencarbonat auf pH 8 eingestellt. Die Phasen werden anschließend getrennt und die wässrige Phase wird nochmals mit 20 ml Dichlormethan extrahiert. Die organische Phase wird destilliert. Man erhält 2,4 g N-Benzyl-2,2-difluorethanamin. Das entspricht einer Ausbeute von 66,2 % bezogen auf eingesetztes 2,2-Difluor-1-chlorethan.
NMR ¹H (CDCl₃): 7,24 - 7,35 (m, 5H), 5,84 (tt, 1 H), 3,84 (s, 2H), 2,95 (dt, 2H)

### Beispiel 1.3

5 g (49,7 mmol) 2,2-Difluor-1-chlorethan und 11,3 g (105 mmol) Benzylamin werden 16 Stunden bei einer Innentemperatur von 120°C in einem Autoklaven erhitzt. Anschließend werden 100 g 1N Salzsäure zugesetzt und das Lösungsmittel im Vakuum entfernt. Der Rückstand wird in 50 ml Wasser und 50 ml Dichlormethan aufgenommen und mit festem Natriumhydrogencarbonat auf pH 8 eingestellt. Die Phasen werden anschließend getrennt und die wässrige Phase wird nochmals mit 20 ml Dichlormethan extrahiert. Die organische Phase wird destilliert. Man erhält 3,5 g N-Benzyl-2,2-difluorethanamin. Das entspricht einer Ausbeute von 41 % bezogen auf eingesetztes 2,2-Difluor-1-chlorethan.
NMR ¹H (CDCl₃): 7,24 - 7,35 (m, 5H), 5,84 (tt, 1 H), 3,84 (s, 2H), 2,95 (dt, 2H)

### Beispiel 1.4

10 g (92 mmol) 2,2-Difluor-1-chlorethan, 5 g (46 mmol) Benzylamin und 5,2 g (51 mmol) Triethylamin werden in 18,7 g N,N-Dimethylacetamid 6 Stunden bei einer Innentemperatur von 120°C in einem Autoklaven erhitzt. Anschließend werden 100 g 1N Salzsäure zugesetzt und das Lösungsmittel im Vakuum entfernt. Der Rückstand wird in 30 ml Dichlormethan aufgenommen und mit festem Natriumhydrogencarbonat auf pH 8 eingestellt. Die Phasen werden anschließend getrennt und die wässrige Phase wird nochmals mit 20 ml Dichlormethan extrahiert. Die organische Phase wird destilliert. Man erhält 7,1 g N-Benzyl-2,2-difluorethanamin. Das entspricht einer Ausbeute von 90 % bezogen auf Benzylamin.

### Beispiel 1.5

10 g (92 mmol) 2,2-Difluor-1-chlorethan, 5 g (46 mmol) Benzylamin und 5,2 g (51 mmol) Triethylamin werden in 20,6 g N-Methylpyrrolidon 6 Stunden bei einer Innentemperatur von 120°C in einem Autoklaven erhitzt. Anschließend werden 100 g 1N Salzsäure zugesetzt und das Lösungsmittel im Vakuum entfernt. Der Rückstand wird in 30 ml Dichlormethan aufgenommen und mit festem Natriumhydrogencarbonat auf pH 8 eingestellt. Die Phasen werden anschließend getrennt und die wässrige Phase wird nochmals mit 20 ml Dichlormethan extrahiert. Die organische Phase wird destilliert. Man erhält 7,3 g N-Benzyl-2,2-difluorethanamin. Das entspricht einer Ausbeute von 93 % bezogen auf Benzylamin.
NMR ¹H (CDCl₃): 7,24 - 7,35 (m, 5H), 5,84 (tt, 1 H), 3,84 (s, 2H), 2,95 (dt, 2H)

### Beispiel 1.6

10 g (92 mmol) 2,2-Difluor-1-chlorethan, 5 g (46 mmol) Benzylamin, 1 g Kaliumbromid und 5,2 g (51 mmol) Triethylamin werden in 20,6 g N-Methylpyrrolidon 6 Stunden bei einer Innentemperatur von 120°C in einem Autoklaven erhitzt. Anschließend werden 100 g 1N Salzsäure zugesetzt und das Lösungsmittel im Vakuum entfernt. Der Rückstand wird in 30 ml Dichlormethan aufgenommen und mit festem Natriumhydrogencarbonat auf pH 8 eingestellt. Die Phasen werden anschließend getrennt und die wässrige Phase wird nochmals mit 20 ml Dichlormethan extrahiert. Die organische Phase wird destilliert. Man erhält 7,4 g N-Benzyl-2,2-difluorethanamin. Das entspricht einer Ausbeute von 94 % bezogen auf Benzylamin.
NMR ¹H (CDCl₃): 7,24 - 7,35 (m, 5H), 5,84 (tt, 1 H), 3,84 (s, 2H), 2,95 (dt, 2H)

### Beispiel 1.7

Wie Beispiel 1.6. Es wird anstelle von 1 g Kaliumbromid 1,5 g Kaliumjodid eingesetzt. Man erhält N-Benzyl-2,2-difluorethanamin in einer Ausbeute von 95 % bezogen auf Benzylamin.
NMR ¹H (CDCl₃): 7,24 - 7,35 (m, 5H), 5,84 (tt, 1 H), 3,84 (s, 2H), 2,95 (dt, 2H)

### Beispiel 1.8

10 g (92 mmol) 2,2-Difluor-1-chlorethan, 5 g (46 mmol) Benzylamin und 5,2 g (51 mmol) Triethylamin werden in 20 g Wasser 6 Stunden bei einer Innentemperatur von 120°C in einem Autoklaven erhitzt. Die zweiphasige Lösung wird zweimal mit je 50 ml Dichlormethan extrahiert, die vereinigten organischen Phasen werden mit 50 ml Wasser gewaschen und anschließend destilliert. Man erhält 5,2 g N-Benzyl-2,2-difluorethanamin. Das entspricht einer Ausbeute von 66% bezogen auf Benzylamin.
NMR ¹H (CDCl₃): 7,24 - 7,35 (m, 5H), 5,84 (tt, 1 H), 3,84 (s, 2H), 2,95 (dt, 2H)

### Beispiel 1.9

Wie Beispiel 1.5. Die Reaktionsmischung wird 6 h bei einer Innentemperatur von 140°C erhitzt. Man erhält N-Benzyl-2,2-difluorethanamin in einer Ausbeute von 83 % bezogen auf Benzylamin.
NMR ¹H (CDCl₃): 7,24 - 7,35 (m, 5H), 5,84 (tt, 1 H), 3,84 (s, 2H), 2,95 (dt, 2H)

Als Nebenkomponente wird das doppel alkylierte Derivat N-Benzyl-N-(2,2-difluorethyl)-2,2-difluorethanamin isoliert.
NMR ¹H (CDCl₃): 7,25 - 7,37 (m, 5H), 5,73 (tt, 2 H), 3,86 (s, 2H), 3,0 (dt, 4H)

### Beispiel 1.10

768,1 g (7,39 mol) 2,2-Difluor-1-chlorethan und 400 g Benzylamin (3,695 mol) werden 16 Stunden bei einer Innentemperatur von 120°C in einem Autoklaven erhitzt. Anschließend werden 700 g Wasser zugesetzt und die wässrige Phase abgetrennt. Die organische Phase wird bei Normaldruck destilliert und das überschüssige 2,2-Difluor-1-chlorethan abdestilliert. Restliche Spuren von 2,2-Difluor-1-chlorethan werden im Vakuum (200 mbar) abdestilliert. Man erhält als Rückstand 304 g N-Benzyl-2,2-difluorethanamin mit einer Reinheit von 97,2 %. Dies entspricht einer Ausbeute von 93,4 % bezogen auf umgesetztes Benzylamin. Das Benzylaminhydrochlorid in der wässrigen Phase kann durch Zusatz von Natronlauge wieder freigesetzt werden und erneut eingesetzt werden.
NMR ¹H (CDCl₃): 7,24 - 7,35 (m, 5H), 5,84 (tt, 1 H), 3,84 (s, 2H), 2,95 (dt, 2H)

### Beispiel 1.11

757,9 g (7,39 mol) 2,2-Difluor-1-chlorethan und 800 g Benzylamin (7,39 mol) werden 16 Stunden bei einer Innentemperatur von 120°C in einem Autoklaven erhitzt. Anschließend werden 1400 g Wasser zugesetzt und die wässrige Phase abgetrennt. Die organische Phase wird bei Normaldruck destilliert und das überschüssige 2,2-Difluor-1-chlorethan abdestilliert. Restliche Spuren von 2,2-Difluor-1-chlorethan werden im Vakuum (200 mbar) abdestilliert. Man erhält als Rückstand 587 g N-Benzyl-2,2-difluorethanamin mit einer Reinheit von 97,4 %. Dies entspricht einer Ausbeute von 93,4 % bezogen auf umgesetztes Benzylamin. Das Benzylaminhydrochlorid in der wässrigen Phase kann durch Zusatz von Natronlauge wieder freigesetzt werden und erneut eingesetzt werden. In der wässrigen Phase sind weitere 21 g N-Benzyl-2,2-difluorethanamin enthalten, sodaß sich die Ausbeute auf 96 % erhöht.
NMR ¹H (CDCl₃): 7,24 - 7,35 (m, 5H), 5,84 (tt, 1 H), 3,84 (s, 2H), 2,95 (dt, 2H)

### Beispiel 2 - Herstellung von 2,2-Difluor-N-(4-methylbenzyl)ethanamin (Schritt (i))

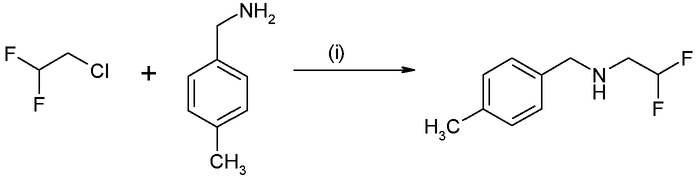

26,2 g (242 mmol) 2,2-Difluor-1-chlorethan und 10 g 4-Methylbenzylamin (80,8 mmol) werden 16 Stunden bei einer Innentemperatur von 120°C in einem Autoklaven erhitzt. Anschließend werden 50 g Wasser zugesetzt und die wässrige Phase abgetrennt. Die wässrige Phase wird nochmals mit 2,2-Difluor-1-chlorethan extrahiert und die vereinigten organischen Phasen werden wie in Beispiel 1.1 beschrieben destilliert. Auch hier kann das 4-Methylbenzylaminhydrochlorid, das sich in der wässrigen Phase befindet, durch Zugabe von Natronlauge wieder in freies 4-Methylbenzylamin überführt werden. Nach Destillation erhält man 4,3 g 2,2-Difluor-N-(4-methylbenzyl)ethanamin. Das entspricht einer Ausbeute von 58 % bezogen auf umgesetztes 4-Methylbenzylamin.
NMR ¹H (CDCl₃): 7,18 (d, 2H), 7,13 (d, 2 H), 5,82 (tt, 1H), 3,78 (s, 2H), 2,93 (dt, 2H), 2,32 (s, 3H)

### Beispiel 3 - Herstellung von 2,2-Difluor-N-(4-chlorbenzyl)ethanamin (Schritt (i))

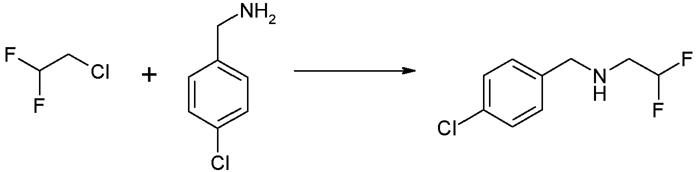

22,4 g (207 mmol) 2,2-Difluor-1-chlorethan und 10 g (69 mmol) 4-Chlorbenzylamin werden 16 Stunden bei einer Innentemperatur von 120°C in einem Autoklaven erhitzt. Anschließend werden 50 g Wasser zugesetzt und die wässrige Phase abgetrennt. Die wässrige Phase wird nochmals mit 2,2-Difluor-1-chlorethan extrahiert und die vereinigten organischen Phasen werden wie in Beispiel 1.1 beschrieben destilliert. Auch hier kann das 4-Chlorbenzylaminhydrochlorid, das sich in der wässrigen Phase befindet, durch Zugabe von Natronlauge wieder in freies 4-Chlorbenzylamin überführt werden. Nach Destillation erhält man 4,25 g 2,2-Difluor-N-(4-chlorbenzyl)ethanamin. Das entspricht einer Ausbeute von 61 % bezogen auf umgesetztes 4-Chlorbenzylamin.
NMR ¹H (CDCl₃): 7,24 - 7,3 (m, 4H), 5,84 (tt, 1H), 3,81 (s, 2H), 2,94 (dt, 2H)

### Beispiel 4 - Herstellung von 2,2-Difluor-N-(4-methoxybenzyl)ethanamin (Schritt (i))

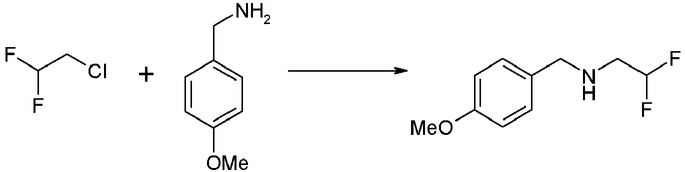

23,15 g (214 mmol) 2,2-Difluor-1-chlorethan und 10 g (71 mmol) 4-Methoxybenzylamin werden 16 Stunden bei einer Innentemperatur von 120°C in einem Autoklaven erhitzt. Anschließend werden 50 g Wasser zugesetzt und die wässrige Phase abgetrennt. Die wässrige Phase wird nochmals mit 2,2-Difluor-1-chlorethan extrahiert und die vereinigten organischen Phasen werden wie in Beispiel 1.1 beschrieben destilliert. Auch hier kann das 4-Methoxybenzylaminhydrochlorid, das sich in der wässrigen Phase befindet, durch Zugabe von Natronlauge wieder in freies 4-Methoxybenzylamin überführt werden. Nach Destillation erhält man 4,93 g 2,2-Difluor-N-(4-methoxybenzyl)ethanamin. Das entspricht einer Ausbeute von 68 % bezogen auf umgesetztes 4-Methoxybenzylamin.
NMR ¹H (CDCl₃): 7,22 (m, 2H), 6,87 (m, 2H), 5,83 (tt, 1H), 3,79 (s, 3H), 3,77 (2H), 2,94 (dt, 2H)

### Beispiel 5 - Herstellung von 2,2-Difluorethylamin (Schritt (ii))

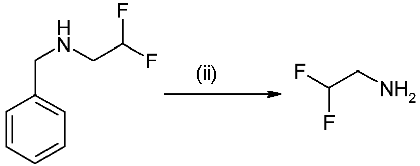

### Beispiel 5.1

In einem Autoklaven werden 280 g (1,62 mol) 2,2-Difluorethylbenzylamin in 1260 mL Toluol gelöst und 7,0 g 5 % Palladium auf Aktivkohle (wasserfeucht, ca. 52 Gew.-% Wasser) zugesetzt. Nach Inertisierung werden 6 bar Wasserstoff aufgedrückt (angelegt) und für 10 Stunden auf 80 °C erwärmt. Nach Filtration des Katalysators wird 2,2-Difluorethylamin destilliert. Es werden 109,8 g 2,2-Difluorethylamin erhalten. Das entspricht einer Ausbeute von 84 % bezogen auf das eingesetzte 2,2-Difluorethylbenzylamin.
NMR ¹H (CDCl₃): 5,5 - 5,9 (m, 1H), 2,94 - 3,1 (m, 2 H), 1,26 (br m, NH2)

## Patentansprüche

1. Ein Verfahren zur Herstellung von 2,2-Difluorethylamin der Formel (I)
CHF₂CH₂NH₂ (I)
umfassend die Schritte (i) und (ii):
Schritt (i): Umsetzung von 2,2-Difluor-1-halogenethan der Formel (II)
CHF₂-CH₂Hal (II)
mit einer Benzylamin-Verbindung der Formel (III) 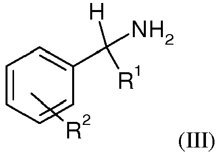 zu einer N-Benzyl-2,2-difluorethanamin-Verbindung der Formel (IV) 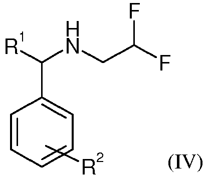 in Gegenwart einer organischen oder anorganischen Base, die in der Lage ist, die frei werdenden Halogenwasserstoff-Verbindungen zu binden, worin in Formel (II) Hal für Chlor, Brom oder Jod steht,
und in den Formeln (III) und (IV)
R¹ für Wasserstoff oder für gegebenenfalls mit Halogen substituiertes C₁-C₁₂ Alkyl steht, und
R² für Wasserstoff, Halogen oder für gegebenenfalls mit Halogen substituiertes C₁-C₁₂-Alkyl oder C₁-C₆-Alkoxy steht;
und
Schritt (ii): Katalytische Hydrierung der im Schritt (i) erhaltenen N-Benzyl-2,2-difluorethanamin-Verbindung der Formel (IV), wodurch 2,2-Difluorethylamin der Formel (I) oder ein Salz davon erhalten wird.

2. Das Verfahren nach Anspruch 1, wobei ein Teil der verwendeten Benzylamin-Verbindung als Base dient, während der andere Teil der Benzylaminverbindung der Formel (III) umgesetzt wird.

3. Das Verfahren nach Anspruch 1, wobei im Schritt (i) als Base Triethylamin eingesetzt wird.

4. Das Verfahren nach Anspruch 2 oder 3, wobei im Schritt (i) die molare Menge an eingesetztem 2,2-Difluorhalogenethan der Formel (II) größer als die molare Menge an Base und umzusetzender Benzylaminverbindung der Formel (III) ist.

5. Das Verfahren nach einem der Ansprüche 1 bis 4, wobei Schritt (i) ohne Lösungsmittel durchgeführt wird.

6. Das Verfahren nach einem der Ansprüche 1 bis 5, wobei Schritt (i) in Gegenwart eines Katalysators durchgeführt wird, der ausgewählt ist aus Alkalibromiden und -jodiden, Ammoniumbromid, Ammoniumjodid, Tetraalkylammoniumbromide, Tetraalkylammoniumjodide, Tetraalkylphosphoniumhalogenide, Tetraarylphosphoniumhalogenide, Tetrakis(dimethylamino)phosphoniumbromid, Tetrakis(diethylamino)phosphoniumbromid, Tetrakis(dipropylamino)phosphoniumchlorid, Tetrakis(dipropylamino)phosphoniumbromid, Tetrakis(dipropylamino)phosphoniumchlorid, Bis(dimethylamino)[(1,3-dimethylimidazolidin-2-yliden)amino]methyliumbromid oder Mischungen davon.

7. Das Verfahren nach einem der Ansprüche 1 bis 6, wobei in den Formeln (III) und (IV) R¹ und R² jeweils für Wasserstoff stehen und in Formel (II) Hal für Chlor steht.

8. Ein Verfahren zur Herstellung von einer N-Benzyl-2,2-difluorethanamin-Verbindung der Formel (IV) 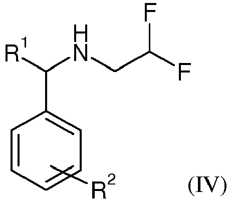 umfassend die Umsetzung von 2,2-Difluor-1-halogenethan der allgemeinen Formel (II)
CHF₂-CH₂Hal (II),
mit einer Benzylamin-Verbindung der Formel (III) 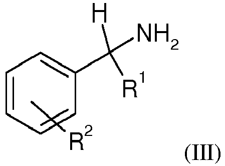 in Gegenwart einer organischen oder anorganischen Base, die in der Lage ist, die frei werdenden Halogenwasserstoff-Verbindungen zu binden,
worin in Formel (II)
Hal für Chlor, Brom oder Jod steht,
und in den Formeln (III) und (IV)
R¹ für Wasserstoff oder für gegebenenfalls mit Halogen substituiertes C₁-C₁₂-Alkyl steht, und
R² für Wasserstoff, Halogen oder für gegebenenfalls mit Halogen substituiertes C₁-C₁₂-Alkyl oder C₁-C₆-Alkoxy steht.

9. Das Verfahren nach Anspruch 8,
worin in den Formeln (III) und (IV)
R¹ für Wasserstoff oder C₁-C₆-Alkyl steht, und
R² für Wasserstoff, Fluor, Chlor, Brom oder Iod, C₁-C₆-Alkyl oder C₁-C₃-Alkoxy oder Methoxy steht.

10. Das Verfahren nach Anspruch 8, worin in den Formeln (III) und (IV) R¹ und R² jeweils für Wasserstoff stehen und in Formel (II) Hal für Chlor steht.

11. Das Verfahren nach einem der Ansprüche 8 bis 10, wobei ein Teil der verwendeten Benzylamin-Verbindung als Base dient, während der andere Teil der Benzylamin-Verbindung der Formel (III) umgesetzt wird.

12. Das Verfahren nach einem der Ansprüche 8 bis 10, wobei als Base Triethylamin eingesetzt wird.

13. Das Verfahren nach einem der Ansprüche 8 bis 12, wobei die molare Menge an eingesetztem 2,2-Difluorhalogenethan der Formel (II) größer als die molare Menge an Base und umzusetzender Benzylamin-Verbindung der Formel (III) ist.

14. Das Verfahren nach einem der Ansprüche 8 bis 13, wobei es ohne Lösungsmittel durchgeführt wird.

15. Das Verfahren nach einem der Ansprüche 8 bis 14, wobei das Verfahren in Gegenwart eines Katalysators durchgeführt wird, der ausgewählt ist aus Alkalibromiden und jodiden, Ammoniumbromid, Ammoniumjodid, Tetraalkylammoniumbromide, Tetraalkylammoniumjodide, Tetraalkylphosphoniumhalogenide, Tetraarylphosphoniumhalogenide, Tetrakis(dimethylamino)phosphoniumbromid, Tetrakis(diethylamino)phosphoniumbromid, Tetrakis(dipropylamino)phosphoniumchlorid, Tetrakis(dipropylamino)phosphoniumchlorid, Tetrakis(dipropylamino)phosphoniumbromid, Bis(dimethylamino)[(1,3-dimethylimidazolidin-2-yliden)amino]methyliumbromid oder Mischungen davon.

## Claims

1. A process for the preparation of 2,2-difluoroethylamine of the formula (I)
CHF₂CH₂NH₂ (I)
comprising the stages (i) and (ii):
stage (i): reaction of 2,2-difluoro-1-halo-ethane of the formula (II)
CHF₂-CH₂Hal (II)
with a benzylamine compound of the formula (III) 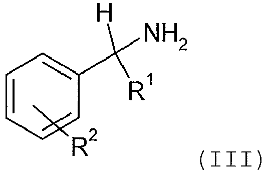 to give an N-benzyl-2,2-difluoroethanamine compound of the formula (IV) 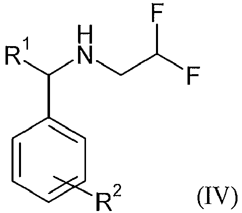 in the presence of an organic or inorganic base which is able to bind the released hydrogen halide compounds,
in which, in formula (II),
Hal is chlorine, bromine or iodine,
and, in the formulae (III) and (IV),
R¹ is hydrogen or optionally halogen-substituted C₁-C₁₂-alkyl, and
R² is hydrogen, halogen or optionally halogen-substituted C₁-C₁₂-alkyl or C₁-C₆-alkoxy;
and
stage (ii): catalytic hydrogenation of the N-benzyl-2,2-difluoroethanamine compound of the formula (IV) obtained in the stage (i), by which 2,2-difluoroethylamine of the formula (I) or a salt thereof is obtained.

2. The process according to Claim 1, in which a portion of the benzylamine compound used acts as base, while the other portion of the benzylamine compound of the formula (III) is reacted.

3. The process according to Claim 1, in which, in stage (i), triethylamine base is used as base.

4. The process according to Claim 2 or 3, in which, in stage (i), the molar amount of 2,2-difluoro-haloethane of the formula (II) used is greater than the molar amount of base and reacting benzylamine compound of the formula (III).

5. The process according to one of Claims 1 to 4, in which stage (i) is carried out without solvent.

6. The process according to one of Claims 1 to 5, in which stage (i) is carried out in the presence of a catalyst chosen from alkali metal bromides and iodides, ammonium bromide, ammonium iodide, tetraalkylammonium bromides, tetraalkylammonium iodides, tetraalkylphosphonium halides, tetraaryl-phosphonium halides, tetrakis(dimethylamino)-phosphonium bromide, tetrakis(diethylamino)-phosphonium bromide, tetrakis(dipropylamino)-phosphonium chloride, tetrakis(dipropylamino)-phosphonium bromide, tetrakis(dipropylamino)-phosphonium chloride, bis(dimethylamino)[(1,3-dimethylimidazolidin-2-ylidene)amino]methylium bromide or mixtures thereof.

7. The process according to one of Claims 1 to 6, in which, in the formulae (III) and (IV), R¹ and _{R}² are each hydrogen and, in formula (II), Hal is chlorine.

8. A process for the preparation of an N-benzyl-2,2-difluoroethanamine compound of the formula (IV) 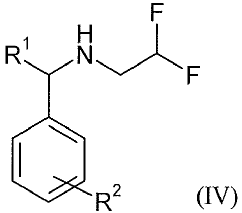 comprising the reaction of 2,2-difluoro-1-haloethane of the general formula (II)
CHF₂-CH₂Hal (II),
with a benzylamine compound of the formula (III) 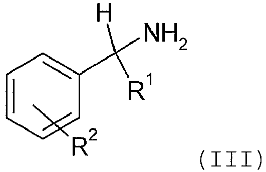 in the presence of an organic or inorganic base which is able to bind the released hydrogen halide compounds,
in which, in formula (II),
Hal is chlorine, bromine or iodine,
and, in the formulae (III) and (IV),
R¹ is hydrogen or optionally halogen-substituted C₁-C₁₂-alkyl, and
R² is hydrogen, halogen or optionally halogen-substituted C₁-C₁₂-alkyl or C₁-C₆-alkoxy.

9. The process according to Claim 8,
in which, in the formulae (III) and (IV),
R¹ is hydrogen or C₁-C₆-alkyl, and
R² is hydrogen, fluorine, chlorine, bromine or iodine, C₁-C₆-alkyl or C₁-C₃-alkoxy or methoxy.

10. The process according to Claim 8, in which, in the formulae (III) and (IV), R¹ and R² are each hydrogen and, in formula (II), Hal is chlorine.

11. The process according to one of Claims 8 to 10, in which a portion of the benzylamine compound used acts as base, while the other portion of the benzylamine compound of the formula (III) is reacted.

12. The process according to one of Claims 8 to 10, in which triethylamine is used as base.

13. The process according to one of Claims 8 to 12, in which the molar amount of 2,2-difluorohaloethane of the formula (II) used is greater than the molar amount of base and reacting benzylamine compound of the formula (III).

14. The process according to one of Claims 8 to 13, where it is carried out without solvent.

15. The process according to one of Claims 8 to 14, where the process is carried out in the presence of a catalyst chosen from alkali metal bromides and iodides, ammonium bromide, ammonium iodide, tetraalkylammonium bromides, tetraalkylammonium iodides, tetraalkylphosphonium halides, tetraarylphosphonium halides, tetrakis(dimethylamino)-phosphonium bromide, tetrakis(diethylamino)-phosphonium bromide, tetrakis(dipropylamino)-phosphonium chloride, tetrakis(dipropylamino)-phosphonium chloride, tetrakis(dipropylamino)-phosphonium bromide, bis(dimethylamino)[(1,3-dimethylimidazolidin-2-ylidene)amino]methylium bromide or mixtures thereof.

## Revendications

1. Procédé pour la préparation de la 2,2-difluoroéthylamine de formule (I)
CHF₂CH₂NH₂ (I)
comprenant les étapes (i) et (ii) :
étape (i) : mise en réaction d'un 2,2-difluoro-1-halogénoéthane de formule (II)
CHF₂-CH₂Hal (II)
avec un composé de type benzylamine de formule (III) 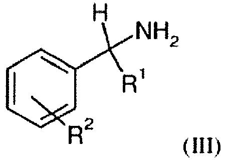 pour l'obtention d'un composé N-benzyl-2,2-difluoroéthanamine de formule (IV) 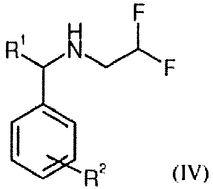 en présence d'une base organique ou inorganique qui est capable de fixer les composés hydracides halogénés libérés, dans la formule (II) Hal représentant le chlore, le brome ou l'iode,
et dans les formules (III) et (IV)
R¹ représentant un atome d'hydrogène ou un groupe alkyle en C₁-C₁₂ éventuellement substitué par halogéno et
R² représentant un atome d'hydrogène ou d'halogène ou un groupe alcoxy en C₁-C₆ ou alkyle en C₁-C₁₂ éventuellement substitué par halogéno ;
et
étape (ii) : hydrogénation catalytique du composé N-benzyl-2,2-difluoroéthanamine de formule (IV) obtenu dans l'étape (i), de sorte qu'on obtient la 2,2-difluoroéthylamine de formule (I) ou un sel de celle-ci.

2. Procédé selon la revendication 1, dans lequel une partie du composé de type benzylamine utilisé sert de base, tandis que l'autre partie du composé de type benzylamine de formule (III) est mise en réaction.

3. Procédé selon la revendication 1, dans lequel on utilise la triéthylamine en tant que base dans l'étape (i).

4. Procédé selon la revendication 2 ou 3, dans lequel la quantité molaire de 2,2-difluorohalogénoéthane de formule (II) utilisé est supérieure à la quantité molaire de base et de composé de type benzylamine de formule (III) à mettre en réaction.

5. Procédé selon l'une quelconque des revendications 1 à 4, dans lequel on effectue l'étape (i) sans solvant.

6. Procédé selon l'une quelconque des revendications 1 à 5, dans lequel on effectue l'étape (i) en présence d'un catalyseur qui est choisi parmi les bromures et iodures de métaux alcalins,
le bromure d'ammonium, l'iodure d'ammonium, les bromures de tétraalkylammonium, les iodures de tétraalkylammonium, les halogénures de tétraalkylphosphonium, les halogénures de tétraarylphosphonium, le bromure de tétrakis(diméthylamino)phosphonium, le bromure de tétrakis(diéthylamino)phosphonium, le chlorure de tétrakis(dipropylamino)phosphonium, le bromure de tétrakis(dipropylamino)phosphonium, le chlorure de tétrakis(dipropylamino)phosphonium, le bromure de bis(diméthylamino)[(1,3-diméthylimidazolidin-2-ylidène)amino]méthylium ou des mélanges de ceux-ci.

7. Procédé selon l'une quelconque des revendications 1 à 6, dans lequel R¹ et R² dans les formules (III) et (IV) représentent chacun un atome d'hydrogène et Hal dans la formule (II) représente un atome de chlore.

8. Procédé pour la préparation d'un composé N-benzyl-2,2-difluoroéthanamine de formule (IV) 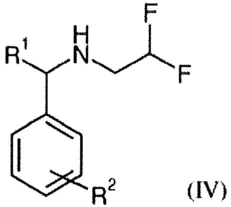 comprenant la mise en réaction d'un 2,2-difluoro-1-halogénoéthane de formule générale (II)
CHF₂-CH₂Hal (II)
avec un composé de type benzylamine de formule (III) 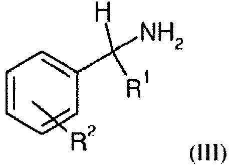 en présence d'une base organique ou inorganique qui est capable de fixer les composés hydracides halogénés libérés,
dans la formule (II)
Hal représentant le chlore, le brome ou l'iode,
et dans les formules (III) et (IV)
R¹ représentant un atome d'hydrogène ou un groupe alkyle en C₁-C₁₂ éventuellement substitué par halogéno et
R² représentant un atome d'hydrogène ou d'halogène ou un groupe alcoxy en C₁-C₆ ou alkyle en C₁-C₁₂ éventuellement substitué par halogéno.

9. Procédé selon la revendication 8,
dans lequel dans les formules (III) et (IV) R¹ représente un atome d'hydrogène ou un groupe alkyle en C₁-C₆ et
R² représente un atome d'hydrogène, de fluor, de chlore, de brome ou d'iode, un groupe alkyle en C₁-C₆ ou alcoxy en C₁-C₃ ou méthoxy.

10. Procédé selon la revendication 8, dans lequel R¹ et R² dans les formules (III) et (IV) représentent chacun un atome d'hydrogène et Hal dans la formule (II) représente le chlore.

11. Procédé selon l'une quelconque des revendications 8 à 10, dans lequel une partie du composé de type benzylamine utilisé sert de base, tandis que l'autre partie du composé de type benzylamine de formule (III) est mise en réaction.

12. Procédé selon l'une quelconque des revendications 8 à 10, dans lequel on utilise comme base la triéthylamine.

13. Procédé selon l'une quelconque des revendications 8 à 12, dans lequel la quantité molaire de 2,2-difluorohalogénoéthane de formule (II) utilisé est supérieure à la quantité molaire de base et de composé de type benzylamine de formule (III) à mettre en réaction.

14. Procédé selon l'une quelconque des revendications 8 à 13, celui-ci étant effectué sans solvant.

15. Procédé selon l'une quelconque des revendications 8 à 14, dans lequel on effectue le procédé en présence d'un catalyseur qui est choisi parmi les bromures et iodures de métaux alcalins,
le bromure d'ammonium, l'iodure d'ammonium, les bromures de tétraalkylammonium, les iodures de tétraalkylammonium, les halogénures de tétraalkylphosphonium, les halogénures de tétraarylphosphonium, le bromure de tétrakis(diméthylamino)phosphonium, le bromure de tétrakis(diéthylamino)phosphonium, le chlorure de tétrakis(dipropylamino)phosphonium, le chlorure de tétrakis(dipropylamino)phosphonium, le bromure de tétrakis(dipropylamino)phosphonium, le bromure de bis(diméthylamino)[(1,3-diméthylimidazolidin-2-ylidène)amino]méthylium ou des mélanges de ceux-ci.
